# EUROPEAN PATENT APPLICATION

(11) **EP 4 702 917 A2**
(43) Date of publication of application: **04.03.2026**
(21) Application number: 25198802.8
(22) Date of filing: 28.08.2025
(51) Int. Cl.: A61B 5/01, A61B 5/0507, A61B 5/00

(54) **APPARATUS FOR NON-INVASIVELY DETERMINING DEEP TISSUE TEMPERATURE USING MICROWAVE RADIOMETRY**

(30) Priority: 28.08.2024 US 202463688040 P
(71) Applicant: Brain Temp, Inc., Bethlehem Pennsylvania 18020 (US)
(72) Inventor: ALLISON, Robert, Rancho Palos Verdes, CA (US)
(74) Representative: IK-IP LTD

(57) **Abstract**

The present disclosure provides a sensor assembly for non-invasive temperature measurement using microwave radiometry. The assembly comprises a circuit board with a thermistor, capacitor, and inductor, where the inductor electrically isolates microwave signals from temperature sensing signals. A coaxial connector is positioned at one end of the circuit board with an antenna aperture at the opposite end. A shield layer extends over the circuit board with a sensor region, transition region, and extended region that are offset from the circuit board components. The shield layer is oriented at an angle relative to the circuit board. The inductor utilizes parasitic capacitance to improve antenna impedance matching, while the capacitor provides microwave signal isolation for the thermistor to prevent interference with signal detection.

## Description

### CROSS-REFERENCE TO RELATED APPLICATIONS

This application claims the benefit of U.S. Application No. 63/688,040, titled APPARATUS FOR NON-INVASIVELY DETERMINING DEEP TISSUE TEMPERATURE USING MICROWAVE RADIOMETRY, filed August 28, 2024, which is hereby incorporated by reference in its entirety.

### FIELD OF DISCLOSURE

The present disclosure relates to non-invasive temperature measurement systems, and more particularly to an apparatus for non-invasively determining deep tissue temperature using microwave radiometry with an integrated sensor assembly comprising a microwave antenna and thermistor configured for brain temperature monitoring.

### BACKGROUND

Accurate measurement of deep tissue temperature, particularly brain temperature, presents significant challenges in medical diagnostics and patient monitoring. Traditional temperature measurement methods rely on surface measurements or invasive procedures that may not accurately reflect the temperature of internal tissues. Surface temperature measurements, such as those obtained from the skin, often differ substantially from the temperature of underlying tissues due to thermal gradients and environmental factors.

Brain temperature monitoring is of particular clinical interest because cerebral temperature serves as an indicator of neurological function and metabolic activity. However, the brain's location within the skull makes direct temperature measurement impractical in most clinical scenarios. Existing approaches for brain temperature assessment include invasive methods that require surgical access or indirect methods that may lack precision.

Microwave radiometry offers a non-invasive approach for measuring deep tissue temperature by detecting naturally occurring electromagnetic radiation emitted by tissues. All objects above absolute zero emit electromagnetic radiation, and the intensity of this radiation correlates with temperature. Biological tissues emit microwave radiation in proportion to their temperature, providing a basis for non-invasive temperature measurement.

However, implementing microwave radiometry for clinical temperature measurement faces several technical challenges. External electromagnetic interference from sources such as wireless communication systems, medical equipment, and environmental sources can contaminate the weak microwave signals emitted by tissues. The sensitivity of microwave detection systems to such interference can compromise measurement accuracy.

Additionally, the design of sensor assemblies for microwave radiometry applications involves balancing multiple competing factors. The sensor must be sufficiently sensitive to detect the weak microwave emissions from tissues while rejecting external interference. The physical design must accommodate the anatomical constraints of the measurement site, particularly when used with pediatric or neonatal patients where size and weight considerations are paramount.

Impedance matching between the sensor and the tissue interface presents another technical consideration. Mismatched impedance can result in signal reflection and reduced measurement accuracy. The integration of reference temperature measurements alongside microwave detection adds complexity to the sensor design while providing calibration capabilities.

Recent developments in wireless communication technology, including the deployment of 5G networks, have introduced new sources of electromagnetic interference in frequency bands previously allocated for satellite communications. This interference can affect microwave radiometry systems operating in overlapping frequency ranges, necessitating improved filtering and signal processing techniques.

### SUMMARY

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

According to an aspect of the present disclosure, a sensor assembly for non-invasive temperature measurement is provided. The sensor assembly comprises a flexible circuit board having a first surface and a second surface opposite the first surface. The flexible circuit board includes a dielectric layer, a first conductive layer disposed on the first surface, and a second conductive layer disposed on the second surface. The first conductive layer includes an antenna aperture configured to receive microwave signals from tissue. The sensor assembly further comprises an inductor electrically connected to the antenna aperture, a thermistor electrically connected to the inductor, and a capacitor electrically connected between the thermistor and a ground connection. The sensor assembly also includes a coaxial connector having a center conductor and an outer conductor, wherein the center conductor is electrically connected to the antenna aperture and the outer conductor is electrically connected to the ground connection. Additionally, the sensor assembly comprises a shield layer positioned above the first surface of the flexible circuit board and configured to reduce electromagnetic interference, and an adhesive layer positioned on the second surface of the flexible circuit board and configured to adhere the sensor assembly to skin.

According to other aspects of the present disclosure, the sensor assembly may include one or more of the following features. The flexible circuit board may have a thickness of approximately 0.006 inches. The shield layer may comprise aluminum foil having a thickness of approximately 0.002 inches. The shield layer may be oriented at an angle relative to the flexible circuit board such that a maximum shielding area is provided in directions of highest antenna sensitivity to electromagnetic interference. The shield layer may have a square shape with sides of approximately 1.5 inches. The sensor assembly may further comprise a foam spacer positioned between the shield layer and the flexible circuit board, wherein the foam spacer comprises closed cell polyethylene foam. The foam spacer may have a thickness of approximately one-sixteenth of an inch. The inductor may have a self-resonant frequency, and a parasitic capacitance of the inductor at the self-resonant frequency may be configured to provide impedance matching for the antenna aperture. The second conductive layer may include an aperture positioned beneath the center conductor of the coaxial connector, wherein the aperture is sized to optimize capacitance between the center conductor and the second conductive layer for impedance matching. The coaxial connector may comprise a push-on connector configured to rotate without disconnecting. The sensor assembly may further comprise a gasket positioned around the coaxial connector to prevent liquid ingress. The adhesive layer may comprise a medical-grade adhesive configured for skin contact. The flexible circuit board may have dimensions of approximately 0.65 inches by 0.96 inches.

According to another aspect of the present disclosure, a radiometer system for measuring deep tissue temperature is provided. The radiometer system comprises a sensor assembly as described above, a switch module electrically connected to the sensor assembly via the coaxial connector, and a radiometer electrically connected to the switch module. The switch module is configured to alternate between receiving signals from the sensor assembly and signals from a reference source. The radiometer is configured to process the alternating signals to determine a temperature difference between tissue temperature and reference temperature.

According to other aspects of the present disclosure, the radiometer system may include one or more of the following features. The switch module may comprise a reference sensor configured to measure ambient temperature and a reference heater configured to generate a reference signal at a controlled temperature. The reference heater may be controllable to maintain a temperature approximately equal to an expected tissue temperature. The radiometer may comprise a frequency mixer and an oscillator configured to convert received RF signals to an intermediate frequency for improved frequency selectivity and rejection of external RF interference. The radiometer may further comprise a low noise amplifier, a band pass filter, an RF detector, a video amplifier, a synchronous detector, and a low pass filter. The switch module and radiometer may be connected by a coaxial cable. The system may be configured to calculate deep tissue temperature using equations known to those skilled in the art.

According to an aspect of the present disclosure, a sensor assembly for non-invasive temperature measurement is provided. The sensor assembly comprises a flexible circuit board having a first surface and a second surface opposite the first surface. The flexible circuit board includes a dielectric layer, a first conductive layer disposed on the first surface, and a second conductive layer disposed on the second surface, wherein the first conductive layer includes an antenna aperture configured to receive microwave signals from tissue. The sensor assembly further comprises an inductor electrically connected to the antenna aperture, a thermistor electrically connected to the inductor and configured to measure skin temperature, and a capacitor electrically connected between the thermistor and a ground connection. The sensor assembly also includes a coaxial connector having a center conductor and an outer conductor, wherein the center conductor is electrically connected to the antenna aperture and the outer conductor is electrically connected to the ground connection. Additionally, the sensor assembly comprises a shield layer positioned above the first surface of the flexible circuit board and configured to reduce electromagnetic interference, and an adhesive layer positioned on the second surface of the flexible circuit board and configured to adhere the sensor assembly to skin.

According to other aspects of the present disclosure, the sensor assembly may include one or more of the following features. The flexible circuit board may have a thickness of approximately 0.006 inches. The shield layer may comprise aluminum foil having a thickness of approximately 0.002 inches. The shield layer may be oriented at an angle relative to the flexible circuit board such that vertices of the shield layer extend from sides of the antenna aperture to provide maximum shielding area in directions of highest antenna sensitivity to electromagnetic interference. The sensor assembly may further comprise a foam spacer positioned between the shield layer and the flexible circuit board, wherein the foam spacer comprises closed cell polyethylene foam.

According to additional aspects of the present disclosure, the inductor may have a self-resonant frequency, and a parasitic capacitance of the inductor at the self-resonant frequency may be configured to provide impedance matching for the antenna aperture. The second conductive layer may include an aperture positioned beneath the center conductor of the coaxial connector, wherein the aperture is sized to optimize capacitance between the center conductor and the second conductive layer for impedance matching. The coaxial connector may comprise a push-on connector configured to rotate without disconnecting.

According to another aspect of the present disclosure, a radiometer system for measuring deep tissue temperature is provided. The radiometer system comprises a sensor assembly including a flexible circuit board having an antenna aperture configured to receive microwave signals from tissue, an inductor electrically connected to the antenna aperture, a thermistor electrically connected to the inductor and configured to measure skin temperature, and a capacitor electrically connected between the thermistor and a ground connection. The system further includes a switch module electrically connected to the sensor assembly and configured to alternate between receiving signals from the sensor assembly and signals from a reference source. The radiometer system also comprises a radiometer electrically connected to the switch module and configured to process the alternating signals to determine a temperature difference between tissue temperature and reference temperature, and a temperature output calculator configured to calculate deep tissue temperature based on the temperature difference.

According to other aspects of the present disclosure, the radiometer system may include one or more of the following features. The switch module may comprise a reference sensor configured to measure ambient temperature and a reference heater configured to generate a reference signal at a controlled temperature. The reference heater may be controllable to maintain a temperature approximately equal to an expected tissue temperature. The radiometer may comprise a frequency mixer and an oscillator configured to convert received RF signals to an intermediate frequency for improved frequency selectivity and rejection of external RF interference. The radiometer may further comprise a low noise amplifier, a band pass filter, an RF detector, a video amplifier, a synchronous detector, and a low pass filter. The temperature output calculator may be configured to calculate deep tissue temperature using equations known to those skilled in the art.

According to another aspect of the present disclosure, a method for non-invasively measuring deep tissue temperature is provided. The method comprises positioning a sensor assembly on skin, the sensor assembly including an antenna aperture, a thermistor, an inductor electrically connecting the antenna aperture to the thermistor, and a capacitor electrically connected between the thermistor and ground. The method further includes receiving microwave signals from tissue through the antenna aperture, and measuring skin temperature using the thermistor while the inductor provides electrical isolation between microwave signals and temperature sensing signals. The method also comprises alternating between processing signals from the sensor assembly and signals from a reference source using a switch module, determining a temperature difference between tissue temperature and reference temperature using a radiometer, and calculating deep tissue temperature based on the temperature difference.

According to other aspects of the present disclosure, the method may include one or more of the following features. The step of alternating between processing signals from the sensor assembly and signals from a reference source may comprise generating a reference signal using a reference heater maintained at a temperature approximately equal to an expected tissue temperature. The reference heater may be controlled by a servo loop that automatically maintains the reference temperature within predetermined tolerance ranges. The step of determining a temperature difference may comprise converting received RF signals to an intermediate frequency using a frequency mixer and an oscillator for improved frequency selectivity and rejection of external RF interference. The sensor assembly may include a shield layer positioned above the antenna aperture and oriented at an angle relative to a flexible circuit board containing the antenna aperture such that vertices of the shield layer extend from sides of the antenna aperture to provide maximum shielding area in directions of highest antenna sensitivity to electromagnetic interference. The step of calculating deep tissue temperature may comprise using equations known to those skilled in the art.

The forgoing general description of the illustrative embodiments and the following detailed description thereof are merely exemplary aspects of the teachings of this disclosure and are not restrictive.

### BRIEF DESCRIPTION OF FIGURES

Non-limiting and non-exhaustive examples are described with reference to the following figures.
FIG. 1A illustrates a top view of a sensor assembly, according to aspects of the present disclosure.
FIG. 1B illustrates a top view of the sensor assembly with a pull tab, according to aspects of the present disclosure.
FIG. 1C illustrates a side view of the sensor assembly, according to aspects of the present disclosure.
FIG. 1D illustrates a top view of an alternate sensor assembly, according to aspects of the present disclosure.
FIG. 2A illustrates an exploded view of a sensor assembly, according to aspects of the present disclosure.
FIG. 2B illustrates a side view of the exploded view of the sensor assembly of FIG. 2A, according to aspects of the present disclosure.
FIG. 2C illustrates a top view of the exploded sensor, foam spacer and insulation layer of the sensor assembly of FIG. 2A, according to aspects of the present disclosure.
FIG. 2D illustrates a top view of the exploded sensor assembly of FIG. 2A with the sensor and foam spacer assembled, according to aspects of the present disclosure.
FIG. 3 illustrates a cross-sectional view and a top view of the coaxial connector, according to aspects of the present disclosure.
FIG. 4 illustrates a top view of a top layer copper pattern, according to aspects of the present disclosure.
FIG. 5 illustrates a top view of a bottom layer copper pattern of the circuit board assembly, according to aspects of the present disclosure.
FIG. 6 illustrates a circuit diagram combined with both bottom and top layer copper patterns, according to aspects of the present disclosure.
FIG. 7 illustrates a circuit diagram of the radiometer system of FIG. 6, according to aspects of the present disclosure.
FIG. 8A illustrates a block diagram of a temperature measurement system, according to aspects of the present disclosure.
FIG. 8B illustrates a block diagram of the temperature measurement system with a reference heater, according to aspects of the present disclosure.
FIG. 8C illustrates a block diagram of the temperature measurement system with heater control, according to aspects of the present disclosure.
FIG. 9A illustrates a block diagram of a signal processing configuration, according to aspects of the present disclosure.
FIG. 9B illustrates an intermediate frequency block diagram, according to aspects of the present disclosure.
FIG. 10 illustrates a temperature sensor device connected to a radiometer, according to aspects of the present disclosure.
FIG. 11A illustrates a top view of a remote switch, according to aspects of the present disclosure.
FIG. 11B illustrates an exploded view of the remote switch of FIG. 11A, according to aspects of the present disclosure.

### DETAILED DESCRIPTION

The following description sets forth exemplary aspects of the present disclosure. It should be recognized, however, that such description is not intended as a limitation on the scope of the present disclosure. Rather, the description also encompasses combinations and modifications to those exemplary aspects described herein.

The present disclosure relates to apparatus and systems for non-invasive temperature measurement using microwave radiometry techniques. Such apparatus may be configured to determine deep tissue temperature by detecting naturally occurring microwave emissions from biological tissues, providing access to temperature measurements at depths within tissue that conventional surface temperature measurement techniques cannot reach. Microwave radiometry operates on the principle that all objects at temperatures above absolute zero emit electromagnetic radiation, including microwave frequencies, with emission intensity correlating directly with the temperature of the emitting material. The intensity of microwave emissions correlates with the temperature of the emitting material. In biological applications, tissues emit microwave signals that can be detected and analyzed to determine temperature characteristics at various depths within the tissue structure. The frequency range used for such measurements may be selected to provide adequate penetration depth while maintaining sufficient signal strength for accurate temperature determination.

Non-invasive temperature measurement systems using microwave radiometry may include sensor components configured to detect microwave emissions from target tissues and reference measurement capabilities to provide baseline comparisons for enhanced measurement accuracy. The detected microwave signals may be processed through electronic components including amplifiers, filters, and signal processing circuits to extract temperature information. Temperature measurement accuracy may be influenced by electromagnetic interference from external sources, impedance matching between sensor components and target tissues, and thermal noise characteristics. Addressing these factors may involve incorporating shielding structures, impedance matching circuits, and reference signal generation capabilities to improve measurement precision and reliability in clinical applications.

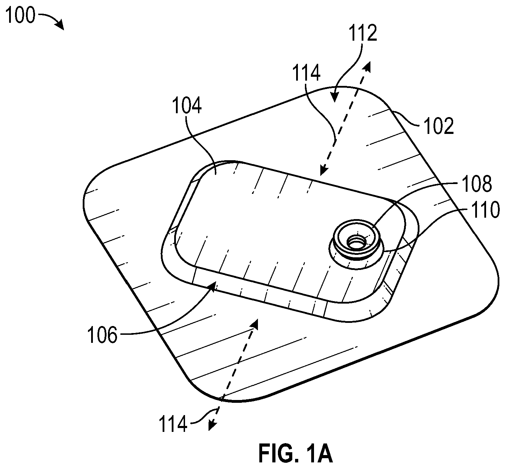

Referring to FIG. 1A, a sensor assembly 100 may be configured for non-invasive temperature measurement applications. The sensor assembly 100 may include a shield layer 102 positioned over a raised portion 104. The raised portion 104 may include a transition portion 106 that connects to a coaxial connector 108. A seal/o-ring 110 may be positioned around the coaxial connector 108 to provide environmental protection. The shield layer 102 may extend along an axis 114 that provides coverage beyond the raised portion 104, and a lower portion 112 may form the base structure of the assembly. The sensor assembly 100 may be configured with overall dimensions of approximately 0.65 inches by 0.96 inches, providing a compact form factor suitable for various measurement applications.

The sensor assembly 100 may incorporate a flexible circuit board having a first surface and a second surface opposite the first surface, with a total thickness of approximately 0.006 inches. This flexible construction may allow the circuit board to conform to curved surfaces such as anatomical contours while maintaining electrical connectivity and measurement functionality. The compact dimensions of approximately 0.65 inches by 0.96 inches may make the sensor assembly 100 particularly suitable for use with neonates, where minimizing bulk while maintaining functionality may benefit patient comfort and measurement accuracy. The shield layer 102 and raised portion 104 may be arranged to allow the coaxial connector 108 to extend through while maintaining shielding coverage around the assembly perimeter, with the transition portion 106 providing connection between the raised portion 104 and surrounding shield layer 102 areas.

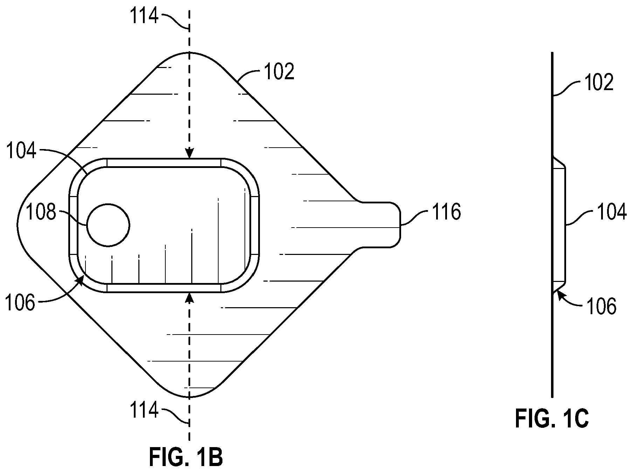

As shown in FIGS. 1B and 1C, the sensor assembly may include a pull tab 116 extending from the shield layer 102. The pull tab 116 may provide a graspable portion of the assembly that facilitates removal without applying stress to adhesive interfaces or underlying sensor components. In some cases, the pull tab 116 may be particularly beneficial in clinical applications where gentle removal may be desired to avoid patient discomfort or skin damage. The pull tab 116 may extend from the shield layer 102 in a direction that provides convenient access while maintaining the overall compact profile of the sensor assembly.

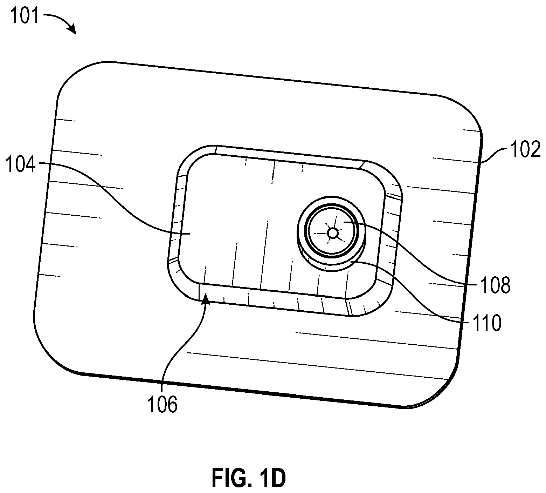

Referring to FIG. 1D, an alternate sensor assembly 101 may provide a different configuration while maintaining similar functional characteristics. The alternate sensor assembly 101 may include the shield layer 102 positioned over the raised portion 104, with the transition portion 106 connecting to the coaxial connector 108. The seal/o-ring 110 may be positioned around the coaxial connector 108 in a similar manner to provide environmental protection. Although the alternate sensor assembly 101 may have the flex circuit oriented parallel to the shielding layer rather than at an angle as in sensor assembly 100, the alternate sensor assembly 101 functions substantially identical to sensor assembly 100 in terms of construction, microwave signal reception, temperature sensing capabilities, and electromagnetic interference rejection. The alternate sensor assembly 101 may demonstrate that various geometric arrangements may be employed while maintaining the fundamental structural relationships between the shield layer 102, raised portion 104, and coaxial connector 108 components.

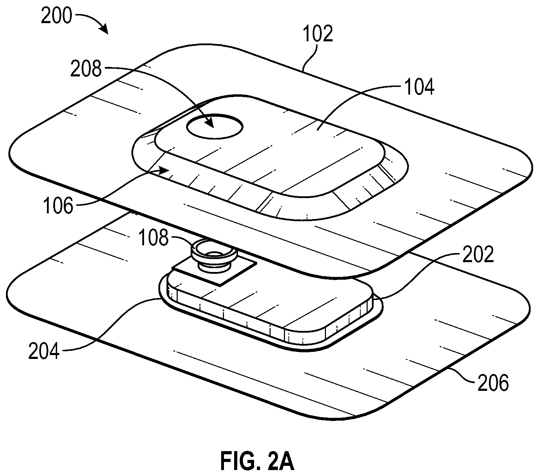

Referring to FIG. 2A, an exploded sensor assembly 200 may illustrate the layered construction of the sensor assembly components. The exploded sensor assembly 200 may include the shield layer 102 positioned at the top of the assembly, with the raised portion 104 connecting to the transition portion 106. A coaxial connector aperture 208 may be formed in the insulation layer to accommodate the coaxial connector 108. The exploded sensor assembly 200 may further include a flex circuit 204 positioned beneath these components, with a foam layer 202 providing spacing between structural elements. An adhesive film 206 may form the bottom layer of the assembly, providing attachment capability to target surfaces. The components may be arranged in a stacked configuration that allows the coaxial connector 108 to extend through while maintaining electromagnetic shielding coverage via the shield layer 102.

The flex circuit 204 may comprise a flexible circuit board with a dielectric layer constructed from Kapton material, providing electrical insulation between conductive layers while maintaining flexibility characteristics. A first conductive layer may be disposed on the first surface and a second conductive layer may be disposed on the second surface, both comprising copper material that provides electrical conductivity. This two-layer copper configuration may replace previous three-layer metal designs, reducing manufacturing complexity while maintaining electrical performance. The total thickness of approximately 0.006 inches and dimensions of approximately 0.65 inches by 0.96 inches may enable the flex circuit 204 to bend and conform to irregular surface geometries without compromising electrical connectivity or structural integrity.

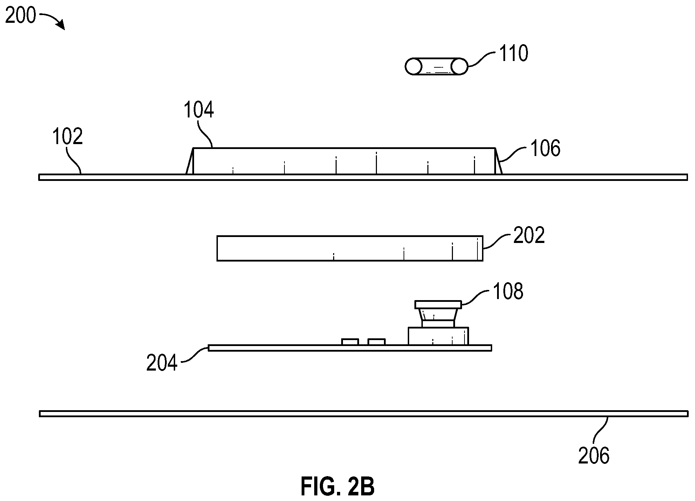

Referring to FIGS. 2B, the exploded sensor assembly 200 may show the relationship between the foam layer 202 and the flex circuit 204 within the overall assembly structure. The foam layer 202 may be positioned beneath the flex circuit 204 and above the adhesive film 206, providing spacing and cushioning between these components. The seal/o-ring 110 may be positioned around the coaxial connector 108 to provide environmental sealing. The adhesive film 206 may be configured to contact a target surface when the sensor assembly is in use, providing attachment capability through medical-grade adhesive materials. The layered arrangement may allow the flex circuit 204 to maintain electrical functionality while accommodating the mechanical requirements of surface attachment and environmental protection.

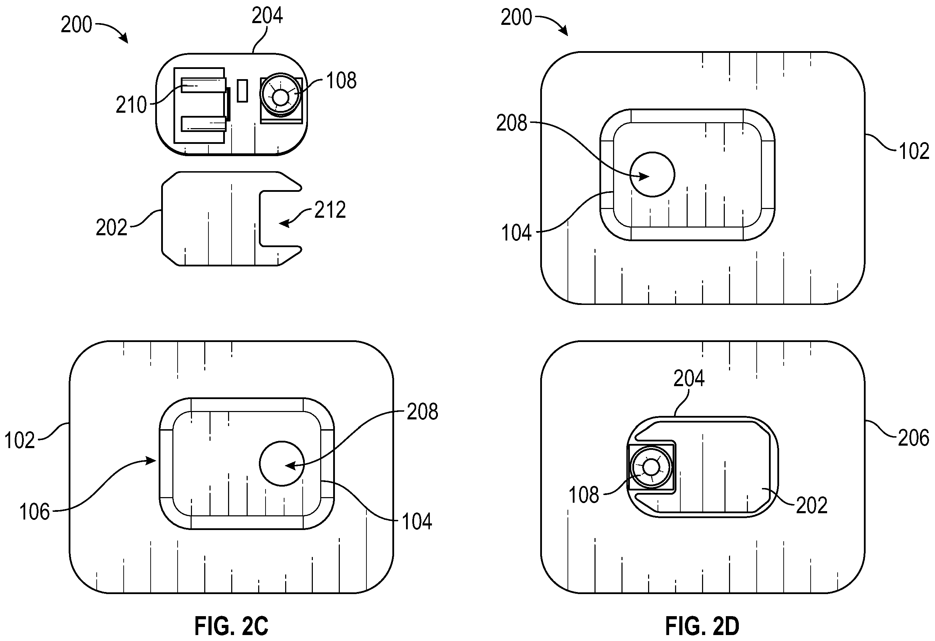

As shown in FIGS. 2C and 2D, the exploded sensor assembly 200 may include an antenna 210 integrated within the flex circuit 204 portion of the assembly. The antenna 210 may be formed as part of the first conductive layer disposed on the first surface of the flex circuit 204, providing microwave signal reception capability. A foam notch 212 may be formed in the foam layer 202 to accommodate the coaxial connector 108 and provide clearance for electrical connections. The coaxial connector aperture 208 may align with the foam notch 212 to allow the coaxial connector 108 to extend through the foam layer 202 and connect to the antenna 210. The antenna 210 may be configured to receive microwave signals from target tissues, with the signals transmitted through the coaxial connector 108 to external processing equipment. The foam layer 202 may provide mechanical support and spacing for the antenna 210 while the foam notch 212 accommodates the physical requirements of the coaxial connector 108 interface.

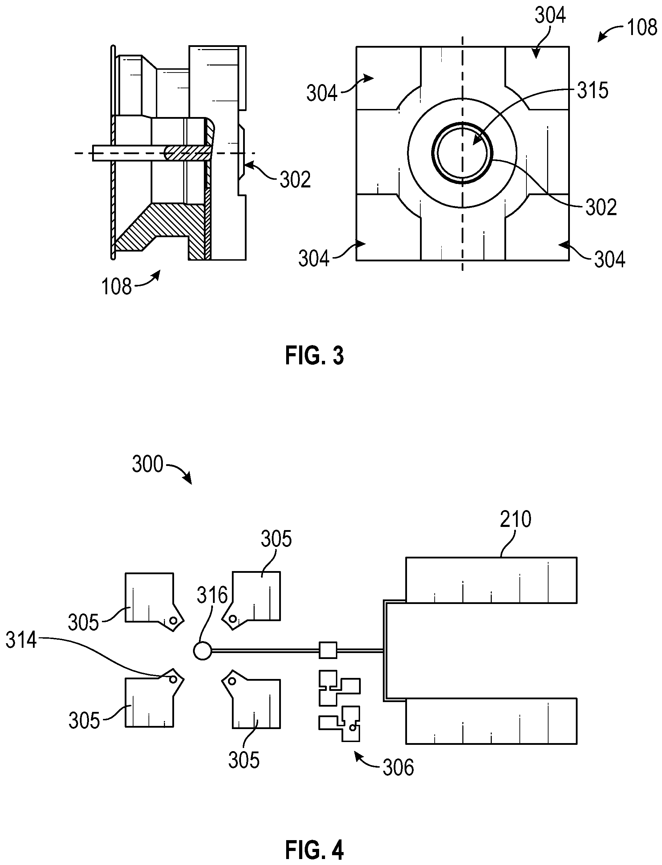

Referring to FIG. 3, the coaxial connector 108 may be illustrated in both cross-sectional and top views to show the mechanical and electrical interface configuration. The cross-sectional view may show the internal structure of the coaxial connector 108, including the center conductor and outer conductor arrangement that provides controlled impedance signal transmission. The center conductor may extend through the connector body to provide electrical connection to the antenna aperture, while the outer conductor may provide electromagnetic shielding and ground return path functionality. The top view may show the external mounting configuration of the coaxial connector 108, including the connector body dimensions and mounting interface that enables attachment to the flexible circuit board. The coaxial connector 108 may incorporate a push-on design that allows rotational movement without disconnecting, reducing mechanical stress on the sensor assembly during cable manipulation.

The antenna 210 may be configured as an antenna aperture formed within the first conductive layer of the flex circuit 204, comprising a rectangular or chamfered rectangular opening that defines the active reception area for microwave emissions from biological tissues. The antenna aperture may receive microwave signals from tissue positioned adjacent to the sensor assembly 100, with signals transmitted to processing components through the coaxial connector 108. The antenna 210 may integrate within sensor circuitry 300 that provides signal conditioning and electrical interface capabilities, including a conductor contact 302 for electrical connection between the coaxial connector 108 and antenna 210, and multiple board contact solder points 305 positioned around the antenna perimeter for connections to the second conductive layer through plated through holes 314.

With continued reference to FIG. 4, the top layer copper pattern may incorporate an RLC circuit 306 that provides signal conditioning functionality for the antenna 210. The RLC circuit 306 may be positioned along the signal path between the antenna 210 and the coaxial connector 108, providing impedance matching and filtering characteristics for the received microwave signals. The conductor contact 302 may interface with the RLC circuit 306 to enable signal transmission from the antenna aperture to external processing equipment. The board contact solder points 305 may be arranged symmetrically around the antenna 210 to provide uniform grounding characteristics and electromagnetic shielding for the antenna aperture. The plated through holes 314 may be positioned to correspond with the board contact solder points 305, enabling electrical continuity between the conductive layers of the flex circuit 204.

The board contact solder points 305 may be electrically connected to the coaxial connector solder points 304 through a soldering process that establishes reliable electrical continuity between the coaxial connector and the circuit board assembly. During assembly, the coaxial connector 108 may be positioned over the board contact solder points 305 such that the coaxial connector solder points 304 align with corresponding board contact solder points 305 and coaxial connection 315 aligns with center conductor contact 316. Once assembled, the coaxial connection 315 may be in electrical communication with center conductor contact 316 so that coaxial cable 702 may be in electrical communication with flex circuit 204. Solder material may be applied to create metallurgical bonds between the coaxial connector solder points 304 and the board contact solder points 305, forming low-resistance electrical connections that provide both mechanical attachment and electrical continuity. The soldering process may establish multiple redundant ground connections between the outer conductor of the coaxial connector 108 and the grounding structure of the flexible circuit board, enhancing electromagnetic shielding performance and providing reliable signal return paths. The solder joints may be configured to maintain electrical integrity under mechanical stress and environmental conditions encountered during sensor assembly use, ensuring consistent performance of the microwave signal transmission interface between the coaxial connector 108 and the antenna 210.

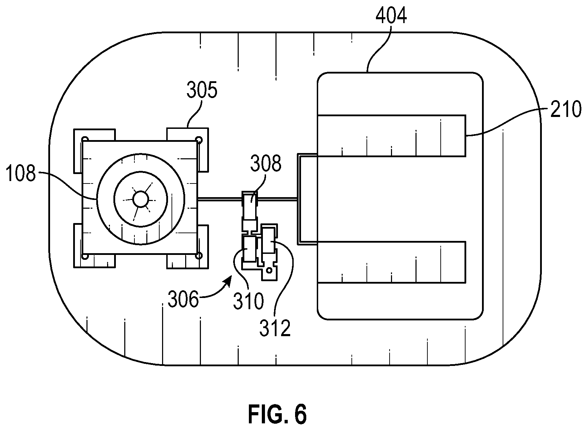

Referring to FIG. 6, a circuit diagram may be illustrated that combines both bottom and top layer copper patterns to show the complete electrical configuration of the sensor assembly. The circuit diagram may show the electrical connections between the antenna aperture, RLC circuit components, and coaxial connector interface, providing a comprehensive view of the signal flow and grounding relationships within the sensor assembly. The combined copper pattern view may illustrate how the first conductive layer and second conductive layer work together to provide signal transmission, grounding, and electromagnetic shielding functions. The circuit diagram may show the inductor 308 electrically connected between the antenna aperture and the thermistor 312, with the capacitor 310 providing a microwave bypass path to ground through the plated through holes 314. The diagram may also illustrate the coaxial connector interface, showing how the center conductor connects to the antenna aperture and how the outer conductor connects to the ground plane formed by the second conductive layer.

The RLC circuit 306 may comprise discrete components including an inductor 308, capacitor 310, and resistor 312 (thermistor). The inductor 308 may electrically connect to the antenna 210 and provide separation between the microwave signal path and temperature sensing components, functioning as a high impedance element at microwave frequencies while allowing direct current flow for temperature sensing. The inductor's self-resonant frequency may contribute to impedance matching characteristics, with parasitic capacitance at the self-resonant frequency configured to provide impedance matching for the antenna aperture. The capacitor 310 may electrically connect between the inductor 308 and ground, providing a microwave frequency path to ground while maintaining isolation for direct current signals. The thermistor may electrically connect to the inductor 308 and may be configured to measure skin temperature while isolated from the microwave signal path, enabling simultaneous microwave signal reception and temperature sensing functionality. The plated hole grounding provided by the RLC circuit 306 may also ground the thermistor 312 and help ensure that the thermistor 312 is at skin temperature since the plated holes 314 are connected to the backside copper plating that contacts the skin through the adhesive layer.

Referring to FIG. 4, the bottom layer copper pattern 400 may form part of the electrical grounding structure for the sensor assembly 100. The bottom layer copper pattern 400 may comprise the second conductive layer disposed on the second surface of the flex circuit 204, providing electrical grounding and signal return paths for the sensor circuitry 300. Multiple plated through holes 314 may be arranged along portions of the bottom layer copper pattern 400 to provide electrical connections between the first conductive layer and the second conductive layer of the flex circuit 204. The plated through holes 314 may enable electrical continuity between the antenna 210 and the grounding structure formed by the bottom layer copper pattern 400. In some cases, the bottom layer copper pattern 400 may provide electromagnetic shielding characteristics that complement the shield layer 102 positioned above the flex circuit 204.

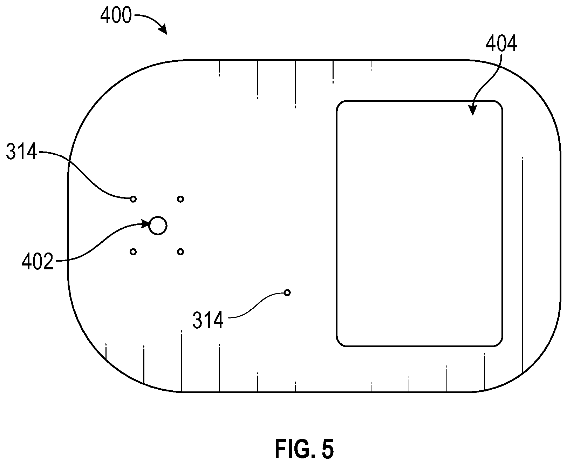

Referring to FIG. 5, the bottom layer copper pattern 400 may form part of the electrical grounding structure for the sensor assembly 100. The bottom layer copper pattern 400 may comprise the second conductive layer disposed on the second surface of the flex circuit 204, providing electrical grounding and signal return paths for the sensor circuitry 300. Multiple plated through holes 314 may be arranged along portions of the bottom layer copper pattern 400 to provide electrical connections between the first conductive layer and the second conductive layer of the flex circuit 204. The plated through holes 314 may enable electrical continuity between the antenna 210 and the grounding structure formed by the bottom layer copper pattern 400. In some cases, the bottom layer copper pattern 400 may provide electromagnetic shielding characteristics that complement the shield layer 102 positioned above the flex circuit 204.

With continued reference to FIG. 5, an access aperture 404 may be located on another portion of the bottom layer copper pattern 400 to provide a window in the bottom copper layer through which the antenna 210 captures radiation from tissue. The access aperture 404 may function as an electromagnetic window that allows microwave emissions from biological tissues to reach the antenna aperture while maintaining the overall grounding characteristics of the bottom layer copper pattern 400. The plated through holes 314 may be positioned around the access aperture 404 to provide ground connections for the capacitor 310 and coaxial connector 108, maintaining electrical connectivity between the conductive layers of the flex circuit 204. The access aperture 404 may be sized and positioned to accommodate the antenna's radiation capture requirements while minimizing disruption to the electromagnetic characteristics of the bottom layer copper pattern 400.

The inductor 308 may electrically connect to the antenna aperture through conductive traces formed within the first conductive layer, with its self-resonant frequency determined by the combination of inductance value and inherent parasitic capacitance. This parasitic capacitance at the self-resonant frequency may provide impedance matching for the antenna aperture, compensating for capacitive or inductive reactances that could degrade signal transmission efficiency. The thermistor may electrically connect to the inductor 308 through conductive traces and may be configured to measure skin temperature by exhibiting resistance changes corresponding to temperature variations. When the sensor assembly 100 is applied to a target surface through the adhesive film 206, the electrical connection between thermistor and inductor may allow direct current flow for temperature measurements while maintaining isolation from microwave frequency signals. The capacitor 310 may electrically connect between the thermistor and ground connection provided by the bottom layer copper pattern 400, functioning as a microwave bypass element that prevents microwave frequency signals from affecting thermistor measurements while allowing independent temperature sensing operation. The capacitor 310 may require a close-by ground connection through the plated through holes 314 to ensure effective microwave bypassing performance.

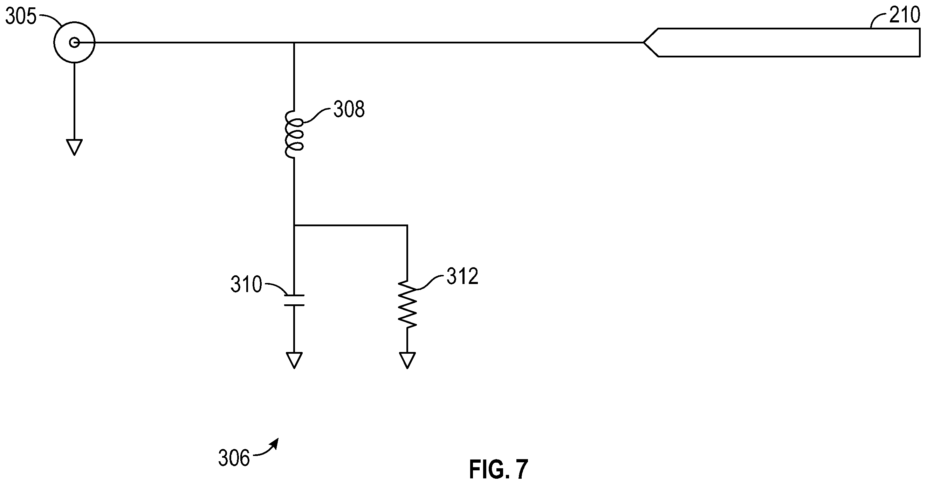

Referring to FIG. 7, a circuit diagram of the radiometer system may illustrate the complete signal processing chain from the sensor assembly through the radiometer components to the temperature output. The circuit diagram may show the electrical connections between the sensor assembly, switch module, and radiometer processing components, providing a comprehensive view of the system architecture and signal flow. The diagram may illustrate how the microwave signals received by the sensor assembly are routed through the switch module to alternate between sensor measurements and reference measurements. The radiometer circuit diagram may show the signal processing components including the low noise amplifier, frequency mixer, oscillator, band pass filter, RF detector, video amplifier, synchronous detector, and low pass filter arranged in a sequential processing chain. The diagram may also show the reference signal generation components including the reference sensor and reference heater that provide controlled temperature references for differential measurement techniques.

The coaxial connector may comprise a center conductor and outer conductor arranged in a coaxial configuration that provides signal transmission capability between the sensor assembly and external processing equipment. The center conductor may electrically connect to the antenna aperture through a conductor contact that provides electrical continuity, maintaining impedance matching characteristics that optimize signal transmission efficiency. The center conductor may be soldered to a round solder pad positioned on the first surface of the flex circuit. The outer conductor may electrically connect to the ground connection through multiple connection points that provide electrical continuity to the second conductive layer, attaching to multiple solder pads positioned around the coaxial connector mounting area. These solder pads may connect through plated through holes 314 to the second conductive layer, establishing a low-impedance ground return path and providing redundant grounding paths that enhance electromagnetic shielding characteristics. The coaxial connector may require close-by ground connections through the plated through holes 314 to maintain proper impedance matching and signal integrity.

The coaxial connector may comprise a push-on connector configured to rotate without disconnecting from the sensor assembly. The push-on connector design may provide mechanical attachment through a spring-loaded or interference fit mechanism that maintains electrical contact while allowing rotational movement. The rotational capability may alleviate torque applied to the connection by external cables or equipment, reducing mechanical stress on the sensor assembly and adhesive attachment to target surfaces. In some cases, the push-on connector may incorporate a captive mechanism that prevents accidental disconnection while permitting rotation about the connector axis. The rotational freedom may accommodate various cable orientations and equipment positioning without applying lateral forces that could cause the sensor assembly to detach from the target surface.

A gasket may be positioned around the coaxial connector to prevent liquid ingress into the sensor assembly. The gasket may comprise an elastomeric material such as rubber or silicone that provides sealing capability between the coaxial connector and the surrounding sensor assembly structure. The gasket may be configured as an O-ring or similar sealing element that compresses between mating surfaces to form a liquid-tight seal around the coaxial connector interface. In some cases, the gasket may comprise a black elastomeric material that provides visual contrast for inspection purposes while maintaining sealing performance under various environmental conditions. The gasket may be positioned within a groove or recess formed around the coaxial connector mounting area, ensuring proper compression and sealing when the connector is installed.

The second conductive layer may include an aperture 402 positioned beneath the center conductor of the coaxial connector to optimize impedance matching characteristics. The aperture may be specifically sized to reduce capacitance between the center conductor solder pad and the ground plane formed by the second conductive layer. The center conductor solder pad may have a diameter that is large relative to the transmission line circuitry on the flex circuit, potentially introducing capacitive discontinuities that could degrade impedance matching performance. The aperture in the second conductive layer may reduce the capacitance between the solder pad and the ground plane by removing conductive material from the area directly beneath the center conductor connection. The size and shape of the aperture may be configured to adjust the capacitance to a value that optimizes the overall impedance matching circuit for the antenna and coaxial connector interface.

The shield layer may be positioned above the first surface of the flexible circuit board and configured to reduce electromagnetic interference affecting microwave signal reception and temperature measurement accuracy. The shield layer may comprise aluminum foil material with a thickness of approximately 1-2 thousandths of an inch, providing electromagnetic shielding characteristics while maintaining flexibility for conforming to curved surfaces. The aluminum foil may be molded to fit over the antenna board, creating a three-dimensional structure with directional shielding coverage around the antenna aperture and associated circuitry. The shield layer may have a square shape with sides of approximately 1.5 inches, providing coverage area that extends beyond the underlying flexible circuit board and antenna components while maintaining a compact footprint suitable for various measurement applications including neonatal temperature monitoring.

The shield layer may be oriented at an angle relative to the flexible circuit board such that vertices of the shield layer extend from sides of the antenna aperture to provide maximum shielding area in directions of highest antenna sensitivity to electromagnetic interference. The antenna aperture may exhibit directional sensitivity characteristics that result in preferential reception of electromagnetic signals from certain orientations. The angled orientation may position the largest shielding coverage area in directions where the antenna demonstrates highest sensitivity to external interference sources. The 45-degree angular relationship may position the shield layer such that the longest shielding distances are provided along axes where the antenna exhibits maximum sensitivity to external electromagnetic fields, enabling the sensor assembly to maintain compact dimensions while achieving electromagnetic interference reduction performance. The shield layer may incorporate a pull tab that extends from the aluminum foil material to provide an easy grip region for removal without damaging fragile patient skin, allowing healthcare providers to remove the sensor assembly by gripping the extended portion rather than manipulating the adhesive bond.

The molded aluminum foil construction of the shield layer may provide electromagnetic interference reduction through multiple mechanisms including reflection, absorption, and field redirection of incident electromagnetic radiation. The conductive properties of the aluminum material may cause incident electromagnetic fields to induce surface currents within the shield layer, with the resulting electromagnetic fields opposing and canceling portions of the incident radiation. The three-dimensional molded structure may provide shielding coverage from multiple angles and directions, reducing the likelihood that electromagnetic interference may reach the antenna aperture through gaps or openings in the shielding structure. In some cases, the molded aluminum foil shield layer may function as a Faraday cage structure that encloses the antenna and associated circuitry, providing comprehensive electromagnetic interference protection while maintaining access for the coaxial connector interface.

A foam spacer may be positioned between the shield layer and the flexible circuit board to provide mechanical support and electrical insulation for the sensor assembly components. The foam spacer may comprise closed cell polyethylene foam material that provides both mechanical and electrical insulation properties, with low dielectric constant characteristics that minimize interference with microwave signal propagation through the antenna aperture. The closed cell structure may prevent moisture absorption that could affect electrical properties over time and provides chemical stability and biocompatibility suitable for medical applications. The foam spacer may have a thickness of approximately one-sixteenth of an inch, providing sufficient spacing to maintain proper electromagnetic isolation between the shield layer and antenna aperture while minimizing the overall profile thickness of the sensor assembly. This thickness may prevent the shield layer from approaching too closely to the antenna aperture, which could cause electromagnetic coupling effects that degrade antenna performance.

The foam spacer may be positioned over the antenna aperture to provide localized insulation and spacing in the region where electromagnetic field interactions between the shield layer and antenna structure are most pronounced, concentrating the insulation effect where electromagnetic coupling could most significantly affect measurement performance. The closed cell structure of the polyethylene foam material may provide dimensional stability that maintains consistent spacing between the shield layer and flexible circuit board over extended periods of use, resisting compression set that could reduce effective thickness under sustained loading. The polyethylene material may exhibit elastic recovery characteristics allowing the foam spacer to return to original thickness after temporary compression, maintaining consistent electromagnetic spacing even after repeated handling or installation cycles. The closed cell foam structure may also provide thermal insulation properties that reduce heat transfer between the shield layer and underlying sensor components, potentially contributing to measurement stability by minimizing thermal gradients within the sensor assembly, enhancing overall measurement accuracy and repeatability across varying environmental conditions and usage scenarios.

An adhesive layer may be positioned on the second surface of the flexible circuit board and configured to adhere the sensor assembly to skin surfaces during temperature measurement applications. The adhesive layer may comprise a medical-grade adhesive configured for skin contact applications, formulated to minimize skin irritation and allergic reactions during prolonged skin contact while providing secure attachment across varying skin types and conditions. The adhesive layer may have a thickness of approximately 4-5 thousandths of an inch, providing sufficient material depth to accommodate minor surface irregularities while maintaining consistent adhesion characteristics. The adhesive layer may cover the entire bottom surface continuously, providing uniform attachment capability across the full contact area and eliminating gaps that could create areas of reduced attachment strength or allow moisture penetration. This continuous coverage may distribute attachment forces uniformly across the sensor assembly footprint, reducing localized stress concentrations and providing consistent thermal contact between the sensor assembly and skin surface.

The adhesive layer may cover the entire bottom surface continuously, providing uniform attachment capability across the full contact area of the sensor assembly. The continuous coverage design may eliminate gaps or discontinuities in the adhesive pattern that could otherwise create areas of reduced attachment strength or allow moisture or contaminants to penetrate between the sensor assembly and skin surface. The continuous adhesive coverage may distribute attachment forces uniformly across the sensor assembly footprint, reducing localized stress concentrations that could cause premature adhesive failure or skin irritation at specific contact points. The uniform coverage pattern may also provide consistent thermal contact between the sensor assembly and skin surface, contributing to measurement accuracy by minimizing thermal variations that could result from non-uniform contact conditions. In some cases, the continuous adhesive coverage may extend across both the flexible circuit board area and any surrounding structural elements, ensuring that the entire sensor assembly perimeter maintains secure attachment to the target skin surface.

The continuous adhesive coverage may extend over portions of the sensor assembly that include antenna components and associated circuitry, requiring consideration of potential electromagnetic effects that the adhesive material may introduce into the measurement system. The adhesive material may exhibit dielectric properties that could affect microwave signal propagation between target tissues and the antenna aperture, with dielectric constant and loss tangent properties incorporated into antenna design calculations to ensure the adhesive layer presence does not significantly alter impedance matching or signal reception characteristics. The medical-grade adhesive may be formulated to provide appropriate adhesion strength for maintaining sensor positioning while allowing controlled removal without excessive force application that could damage fragile skin surfaces, with time-dependent bonding characteristics where initial attachment strength is sufficient for immediate positioning and adhesion strength develops over time for secure long-term attachment. The adhesive layer configuration may accommodate the flexible nature of the underlying circuit board by maintaining adhesion performance even when the sensor assembly conforms to curved or irregular skin surfaces, with the adhesive material exhibiting flexibility characteristics that allow stretching or deforming in coordination with the flexible circuit board without compromising adhesion strength, enabling the sensor assembly to maintain intimate contact with skin surfaces during patient movement or positioning changes.

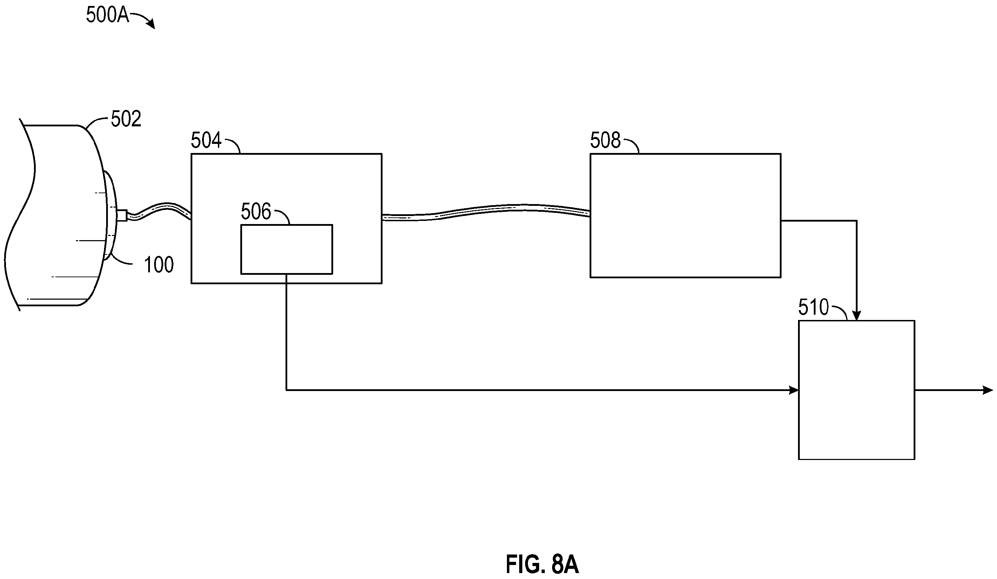

Referring to FIG. 8A, a block diagram 500A may illustrate a temperature measurement system that incorporates the sensor assembly 100 within a comprehensive radiometer configuration. The sensor assembly 100 may connect to a target site 502 that represents the tissue area where temperature measurements may be performed. A switch 504 may be electrically connected to the sensor assembly 100 via the coaxial connector 108, providing signal routing capability between the sensor assembly 100 and downstream processing components. The switch 504 may include a reference sensor 506 that provides reference measurements for comparison with signals received from the sensor assembly 100. A radiometer 508 may be electrically connected to the switch 504, enabling signal processing and analysis of the alternating signals received from the sensor assembly 100 and reference sensor 506. A temperature output calculator 510 may connect to the radiometer 508 to determine temperature measurements based on the processed signals, providing final temperature values that correspond to the target tissue conditions.

The switch 504 may be configured to alternate between receiving signals from the sensor assembly 100 and signals from a reference source provided by the reference sensor 506. The alternating signal configuration may enable the radiometer 508 to process comparative measurements that reduce systematic errors and improve measurement accuracy compared to single-channel measurement approaches. The reference sensor 506 may be configured to measure ambient temperature or other reference conditions that provide baseline measurements for comparison with signals received from the target site 502. The alternating operation of the switch 504 may occur at predetermined intervals or frequencies that allow the radiometer 508 to maintain calibration accuracy while providing continuous temperature monitoring capability. In some cases, the switch 504 may incorporate electronic switching elements that provide rapid alternation between signal sources without introducing significant signal degradation or measurement delays.

With continued reference to FIG. 8A, the radiometer 508 may be configured to process the alternating signals to determine a temperature difference between tissue temperature detected by the sensor assembly 100 and reference temperature measured by the reference sensor 506. The temperature difference calculation may compensate for environmental variations and system drift effects that could otherwise affect measurement accuracy over extended monitoring periods. The radiometer 508 may incorporate signal processing algorithms that account for the timing and characteristics of the alternating signals to extract temperature information from the received microwave emissions. The temperature output calculator 510 may apply mathematical relationships that convert the processed signal differences into absolute temperature values corresponding to the target tissue conditions. The temperature output calculator 510 may incorporate calibration factors and correction algorithms that account for tissue properties, sensor characteristics, and environmental conditions that influence the relationship between detected microwave signals and actual tissue temperatures.

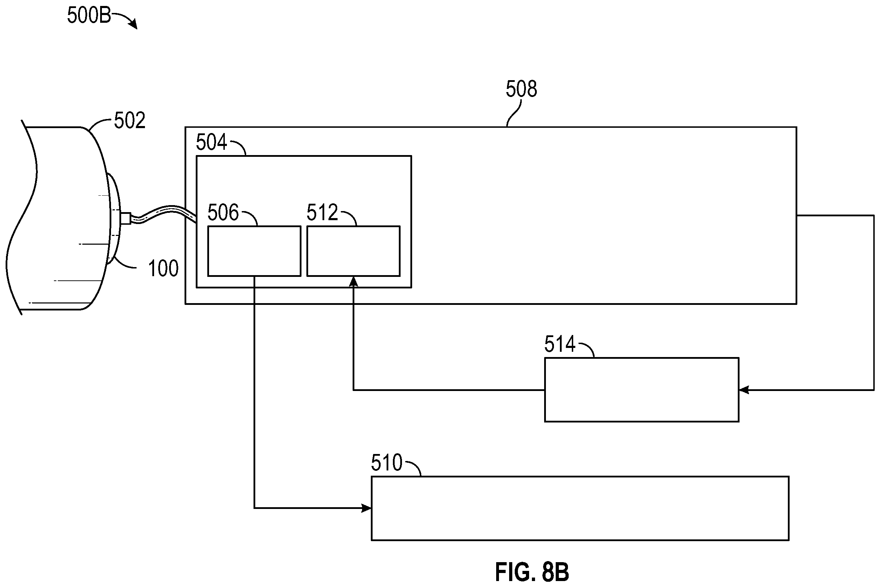

Referring to FIG. 8B, a block diagram 500B may illustrate an enhanced radiometer system configuration that incorporates additional reference signal generation capabilities. The block diagram 500B may include the sensor assembly 100 connected to the target site 502, with the switch 504 providing signal routing between the sensor assembly 100 and the reference sensor 506. A reference heater 512 may be connected to the reference sensor 506 to provide controlled temperature reference signals that enhance measurement accuracy and system calibration. The reference heater 512 may be configured to generate a reference signal at a controlled temperature that corresponds to expected tissue temperature ranges encountered during measurement applications. A heater controller/servo loop 514 may be connected to the reference heater 512 to provide temperature regulation and control functionality that maintains stable reference conditions. The radiometer 508 may process signals received through the switch 504 from both the sensor assembly 100 and the reference heater 512 configuration, enabling enhanced measurement accuracy through controlled reference signal generation.

The reference heater 512 may be controllable to maintain a temperature approximately equal to an expected tissue temperature, reducing impedance mismatch and sensor error that could otherwise affect measurement precision. The controlled temperature operation of the reference heater 512 may provide reference signals that closely match the characteristics of signals received from target tissues, minimizing differential effects that could introduce measurement errors. The heater controller/servo loop 514 may incorporate feedback control mechanisms that monitor the temperature of the reference heater 512 and adjust heating power to maintain stable temperature conditions. The servo loop functionality may compensate for environmental temperature variations and thermal drift effects that could otherwise cause reference signal variations over time. In some cases, the reference heater 512 may be heated to approximately the same temperature as the target tissue to reduce impedance mismatch between the transmitted signal and reflected signal characteristics, minimizing measurement errors that result from signal reflection at the sensor interface.

With continued reference to FIG. 8B, the heater controller/servo loop 514 may provide automatic temperature regulation that maintains consistent reference conditions without requiring manual adjustment during measurement procedures. The servo loop control may incorporate temperature sensing elements that monitor the reference heater 512 temperature and provide feedback signals to the heater controller portion of the system. The heater controller/servo loop 514 may automatically maintain the reference temperature within predetermined tolerance ranges by adjusting electrical power supplied to the reference heater 512 based on the feedback signals, maintaining temperature stability that ensures measurement accuracy. The controlled reference temperature may be set to match expected target tissue temperatures, providing reference signals that exhibit similar electromagnetic characteristics to the signals received from biological tissues. The temperature matching approach may reduce differential signal processing requirements within the radiometer 508, simplifying signal analysis while improving measurement precision through reduced systematic error sources.

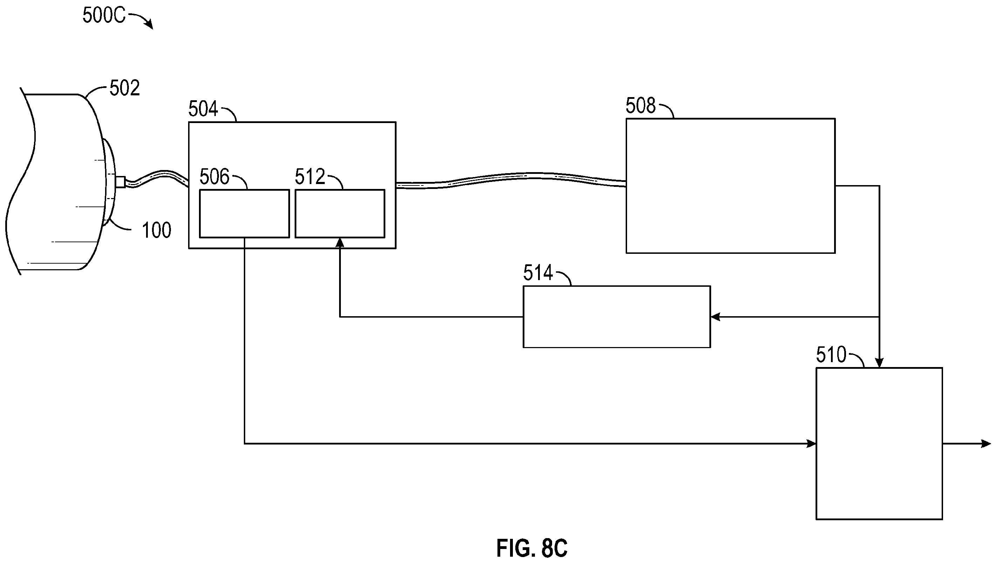

Referring to FIG. 8C, a block diagram 500C may illustrate an alternative radiometer system configuration where the reference heater 512 and heater controller/servo loop 514 may be positioned to provide enhanced reference signal control. The sensor assembly 100 may connect to the target site 502 through the same interface configuration, with the switch 504 providing signal routing between the sensor assembly 100 and reference signal sources. The reference heater 512 may connect directly to the switch 504 in addition to connecting to the heater controller/servo loop 514, providing multiple signal path options for reference signal generation. The reference sensor 506 may connect to the radiometer 508 through the switch 504, enabling the radiometer 508 to process both target signals from the sensor assembly 100 and reference signals from the reference heater 512 and reference sensor 506 combination. The temperature output calculator 510 may receive processed signals from the radiometer 508 and apply calculation algorithms that account for the multiple reference signal sources available through the enhanced system configuration.

The reference heater 512 positioning within the block diagram 500C may provide direct signal input to the switch 504, enabling the switch 504 to alternate between target signals from the sensor assembly 100 and controlled reference signals from the reference heater 512. The direct connection configuration may reduce signal path complexity while maintaining the alternating signal capability that enables differential measurement techniques. The heater controller/servo loop 514 may maintain temperature control of the reference heater 512 independently of the signal routing functions performed by the switch 504, allowing temperature regulation to operate continuously while signal switching occurs at predetermined intervals. The reference sensor 506 may provide additional reference measurements that complement the controlled reference signals from the reference heater 512, enabling multiple reference signal sources that enhance measurement accuracy and system calibration capabilities. In some cases, the multiple reference signal configuration may provide redundant reference measurements that improve system reliability and enable detection of reference signal anomalies that could otherwise affect measurement accuracy.

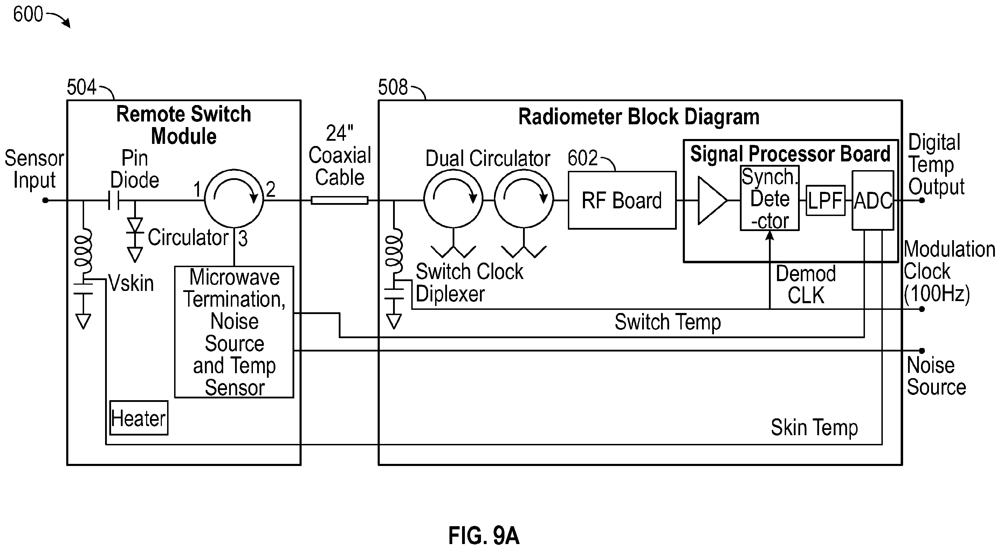

Referring to FIG. 9A, a block diagram 600 may illustrate signal processing enhancements that address electromagnetic interference challenges encountered in radiometer systems. The switch 504 may connect to an intermediate frequency block diagram 602 that provides frequency conversion and filtering capabilities designed to improve signal selectivity and interference rejection. The intermediate frequency block diagram 602 may process signals received from the switch 504 through frequency conversion techniques that translate received microwave signals to intermediate frequency ranges where narrow band filtering may be more practical and effective. The frequency conversion approach may enable the radiometer system to operate in frequency bands that avoid terrestrial interference sources while maintaining sensitivity to microwave emissions from biological tissues. The block diagram 600 configuration may represent an enhanced signal processing architecture that addresses interference challenges that have emerged with the deployment of terrestrial communication systems in frequency bands previously allocated for satellite communications.

The intermediate frequency block diagram 602 may incorporate frequency conversion techniques that translate received signals from the original microwave frequency range to lower intermediate frequencies where filtering and signal processing may be performed with improved selectivity characteristics. The frequency translation process may enable the use of narrow band filtering techniques that provide enhanced rejection of external RF interference sources such as 5G communication systems that operate in portions of the microwave frequency spectrum. The intermediate frequency approach may maintain sensitivity to the desired microwave emissions from target tissues while providing improved discrimination against interference signals that could otherwise degrade measurement accuracy. In some cases, the intermediate frequency block diagram 602 may enable the radiometer system to operate in satellite downlink frequency bands that avoid terrestrial 5G interference frequencies, providing improved measurement performance in environments where terrestrial communication systems may be present.

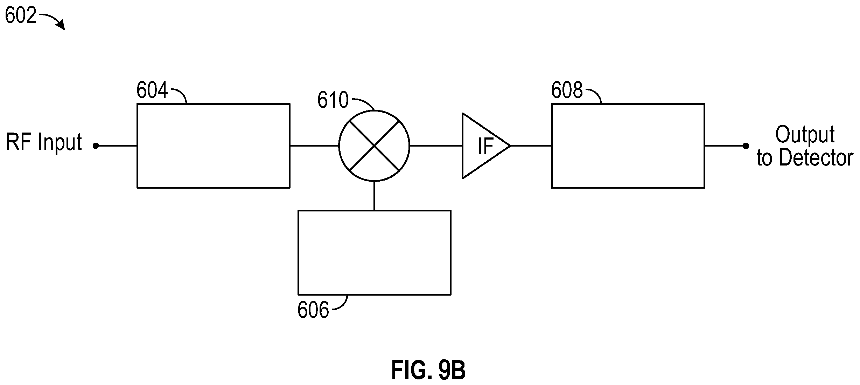

Referring to FIG. 9B, the intermediate frequency block diagram 602 may include specific signal processing components that implement frequency conversion and filtering functions. An RF band pass filter 604 may be positioned at the input stage of the intermediate frequency block diagram 602 to provide initial filtering of signals received from the switch 504. The RF band pass filter 604 may be configured to pass microwave signals within the desired frequency range while attenuating signals outside the target frequency band that could represent interference sources. A frequency mixer 610 may be positioned downstream from the RF band pass filter 604 to provide frequency conversion capability by combining the filtered RF signals with reference signals from a fixed frequency oscillator 606. The frequency mixer 610 may translate the filtered RF signals to intermediate frequency ranges through mixing or heterodyne techniques that enable enhanced signal processing. A fixed frequency oscillator 606 may connect to the frequency mixer 610 to provide stable reference signals at predetermined frequencies that enable consistent frequency conversion performance across varying environmental conditions and system operating parameters.

An IF band pass filter 608 may connect to the fixed frequency oscillator 606 to provide additional filtering of the frequency-converted signals at the intermediate frequency range. The IF band pass filter 608 may be configured with narrow band characteristics that provide enhanced selectivity compared to filtering techniques that may be practical at the original microwave frequencies. The narrow band filtering capability of the IF band pass filter 608 may enable improved rejection of interference signals while maintaining sensitivity to the desired microwave emissions from target tissues. The combination of the RF band pass filter 604, fixed frequency oscillator 606, and IF band pass filter 608 may provide a signal processing chain that improves frequency selectivity and external RF interference rejection compared to direct processing of the original microwave signals. In some cases, the intermediate frequency processing approach may enable the radiometer system to maintain measurement accuracy in environments where external interference sources such as 5G communication systems may be present in portions of the microwave frequency spectrum.

With continued reference to FIG. 9B, the fixed frequency oscillator 606 may provide frequency mixing capability that converts the RF input frequency down to a lower intermediate frequency where narrow band filtering may be more practical and effective. The intermediate frequency may represent the difference between the RF frequency received from the RF band pass filter 604 and the oscillator frequency generated by the fixed frequency oscillator 606. The frequency difference calculation may enable selection of intermediate frequency values that optimize filtering performance while maintaining compatibility with downstream signal processing components. The fixed frequency oscillator 606 may incorporate frequency stability characteristics that ensure consistent frequency conversion performance over temperature variations and aging effects that could otherwise introduce measurement errors. The oscillator frequency selection may be coordinated with the filter characteristics of the RF band pass filter 604 and IF band pass filter 608 to provide optimized overall system performance for the specific frequency bands and interference environments encountered in radiometer applications.

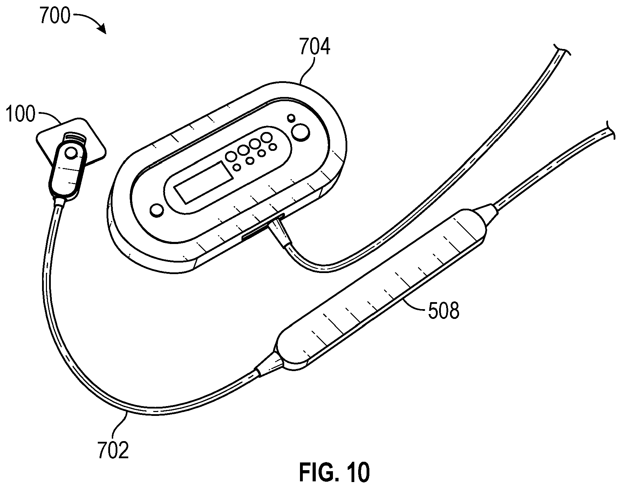

Referring to FIG. 10, a temperature sensor device 700 may illustrate a complete radiometer system implementation that incorporates the sensor assembly 100 within a portable measurement configuration. The temperature sensor device 700 may include the radiometer 508 housed within a portable enclosure that provides environmental protection and user interface capabilities for temperature measurement applications. A coaxial cable 702 may provide electrical connection between the sensor assembly 100 and the radiometer 508, enabling signal transmission while allowing physical separation between the sensor attachment location and the radiometer processing components. The coaxial cable 702 may maintain controlled impedance characteristics that preserve signal integrity during transmission from the sensor assembly 100 to the radiometer 508. A temperature display 704 may be incorporated within the temperature sensor device 700 to provide visual indication of measured temperature values, enabling healthcare providers or researchers to monitor temperature measurements in real-time during measurement procedures.

The coaxial cable 702 may enable flexible positioning of the radiometer 508 relative to the sensor assembly 100 attachment location, accommodating various patient positioning requirements and equipment placement constraints that may be encountered in clinical environments. The cable length and construction may be selected to minimize signal loss while providing sufficient physical separation to avoid interference between the radiometer 508 components and the sensor assembly 100 measurement functions. The coaxial cable 702 may incorporate shielding characteristics that prevent external electromagnetic interference from affecting signal transmission between the sensor assembly 100 and the radiometer 508. The cable connector interfaces may be configured to provide secure electrical connections while allowing for repeated connection and disconnection cycles that may be required during sensor replacement or system maintenance procedures. In some cases, the coaxial cable 702 may incorporate strain relief features that protect the electrical connections from mechanical stress that could result from cable movement or patient positioning changes during measurement procedures.

With continued reference to FIG. 10, the temperature display 704 may be configured to show temperature readings processed by the radiometer 508, providing immediate feedback regarding target tissue temperature conditions. The temperature display 704 may incorporate digital display technology that provides numerical temperature values with precision appropriate for clinical temperature monitoring applications. The display may include units indication and measurement status information that enables healthcare providers to interpret temperature readings and assess measurement validity. The temperature display 704 may incorporate user interface elements such as control buttons or touch screen capability that allow adjustment of measurement parameters and system configuration settings. The display brightness and contrast characteristics may be configured for visibility under various lighting conditions encountered in clinical environments, ensuring that temperature readings remain visible during both normal lighting and reduced lighting conditions that may be present in patient care areas. In some cases, the temperature display 704 may incorporate data logging capabilities that store temperature measurements over time, enabling trend analysis and documentation of temperature variations during extended monitoring periods.

The radiometer may comprise multiple internal components that work together to process microwave signals received from the sensor assembly and convert those signals into temperature measurements. The radiometer components may be configured to handle the alternating signals received through the switch, enabling differential measurement techniques that improve accuracy and reduce systematic errors. The internal architecture of the radiometer may incorporate signal conditioning, amplification, filtering, and detection stages that extract temperature information from the received microwave emissions while rejecting external interference sources that could otherwise degrade measurement performance.

The radiometer may comprise multiple internal components that process microwave signals received from the sensor assembly and convert those signals into temperature measurements. A low noise amplifier may be positioned at the input stage to provide signal amplification while minimizing thermal noise addition that could reduce measurement sensitivity. The amplifier may be configured to operate across the microwave frequency range used for radiometric temperature measurements, providing sufficient gain to raise signal levels above the noise floor of downstream processing components while incorporating automatic gain control features. A frequency mixer may electrically connect to the low noise amplifier to provide frequency conversion capability that translates received RF signals to intermediate frequency ranges where enhanced signal processing may be performed. The frequency mixer may combine amplified RF signals with reference signals generated by an oscillator to produce intermediate frequency outputs representing the difference between input RF frequency and oscillator frequency. An oscillator may be configured to generate stable reference signals that enable consistent frequency conversion operations, providing fixed frequency output signals at predetermined frequencies selected to produce intermediate frequency values optimized for downstream filtering and detection processes.

The combination of the frequency mixer and oscillator may be configured to convert received RF signals to an intermediate frequency for improved frequency selectivity and rejection of external RF interference. The intermediate frequency selection may enable the use of narrow band filtering techniques that provide enhanced discrimination against interference sources such as terrestrial communication systems operating in portions of the microwave spectrum. The frequency conversion approach may allow the radiometer to operate in satellite downlink frequency bands while avoiding terrestrial interference frequencies that have been allocated for commercial communication services. The intermediate frequency processing may maintain sensitivity to microwave emissions from biological tissues while providing improved rejection of external interference sources that could otherwise affect measurement accuracy. The frequency conversion process may enable the radiometer system to adapt to changing interference environments by selecting intermediate frequencies that avoid specific interference sources while maintaining measurement performance.

A band pass filter may be positioned downstream from the frequency mixer to provide selective filtering of intermediate frequency signals while attenuating unwanted frequency components present in the mixer output. The band pass filter may be configured with narrow band characteristics that provide enhanced selectivity at the intermediate frequency compared to filtering techniques practical at original RF frequencies, incorporating multiple filter stages or cascaded filter sections for enhanced selectivity while maintaining acceptable insertion loss characteristics. An RF detector may electrically connect to the band pass filter to convert filtered intermediate frequency signals into baseband signals representing amplitude characteristics of received microwave emissions. The RF detector may incorporate diode detection, logarithmic amplification, or other detection techniques providing linear response to signal amplitude variations while maintaining sensitivity across the dynamic range of signals encountered in radiometric measurements. A video amplifier may be positioned downstream from the RF detector to provide amplification of baseband signals while maintaining bandwidth characteristics needed for processing alternating signals generated by the switch, providing gain adjustment capabilities and offset adjustment features that enable nulling of DC components.

A synchronous detector may be electrically connected to the video amplifier to provide phase-sensitive detection of the alternating signals that correspond to the switching operation between target signals and reference signals. The synchronous detector may receive timing reference signals that correspond to the switch operation, enabling the detector to distinguish between signal components that originate from the target tissue measurements and signal components that originate from the reference measurements. The synchronous detection process may provide enhanced sensitivity to small signal differences between target and reference measurements while rejecting noise components that are not correlated with the switching timing. The synchronous detector may incorporate lock-in amplifier techniques that provide narrow bandwidth detection characteristics, improving signal-to-noise ratio for the differential measurements used to determine temperature differences. The detector output may provide signals that represent the amplitude relationship between target and reference measurements, enabling extraction of temperature information through mathematical processing of the detected signal characteristics.

A low pass filter may be positioned downstream from the synchronous detector to provide filtering of the detected signals while removing high frequency noise components and switching transients that could otherwise affect measurement accuracy. The low pass filter may be configured with cutoff frequency characteristics that preserve the signal components associated with temperature variations while attenuating noise components at frequencies above the measurement bandwidth. The filter characteristics may be selected to provide adequate settling time for the alternating measurements while maintaining sufficient bandwidth to accommodate temperature changes that may occur during measurement procedures. The low pass filter may incorporate multiple filter stages that provide enhanced noise reduction. In some cases, the low pass filter may be implemented using active filter techniques that provide adjustable cutoff frequency characteristics, enabling optimization of the filtering performance for specific measurement applications or environmental noise conditions.

The radiometer components may be configured to process the alternating signals received through the switch to determine temperature differences between tissue temperature measurements and reference temperature measurements. The signal processing chain may maintain the amplitude relationship between the alternating signals while providing amplification, filtering, and detection functions that extract temperature information from the received microwave emissions. The component arrangement may provide multiple stages of signal conditioning that progressively improve signal-to-noise ratio while maintaining sensitivity to the small signal differences that correspond to temperature variations in biological tissues. The radiometer processing may incorporate calibration techniques that account for component characteristics and environmental effects, ensuring that the temperature difference calculations accurately represent the actual temperature conditions at the target tissue locations. The alternating signal processing approach may enable continuous temperature monitoring while providing automatic compensation for system drift effects and environmental variations that could otherwise affect measurement accuracy over extended monitoring periods.

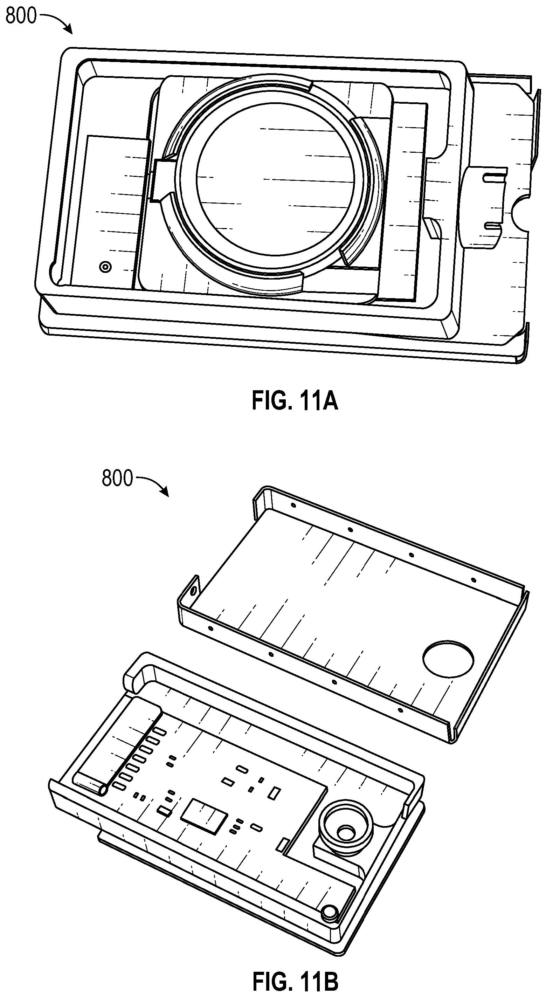

Referring to FIGS. 11A-11B, a remote switch 800, shown in FIG. 9A as switch 504, may illustrate an alternative configuration that demonstrates the integration of temperature measurement components within a compact housing structure. The remote switch 800 may incorporate the temperature calculation methodologies and system operation principles that enable accurate determination of deep tissue temperature through the combination of microwave radiometry and surface temperature measurements. The remote switch 800 may provide a platform for implementing the mathematical relationships that convert detected microwave signals and thermistor measurements into target tissue temperature values that correspond to deep tissue conditions at specified depths within biological tissues.

The temperature output calculator 510 may be configured to implement mathematical algorithms that combine skin temperature measurements obtained from the thermistor with radiometer temperature data derived from microwave signal processing to calculate target tissue temperature values. The calculation methodology may incorporate the relationship between surface temperature conditions and deep tissue temperature characteristics, accounting for thermal gradients and tissue properties that influence heat transfer between different tissue layers. The radiometer 508 may generate radiometer temperature values representing a composite measurement derived from microwave emissions received from multiple tissue layers within the measurement volume of the antenna 210, corresponding to a weighted average of temperature contributions from various tissue depths with weighting factors determined by attenuation characteristics and electromagnetic properties of different tissue types encountered along the microwave signal propagation path.

With continued reference to FIGS. 11A-11B, the system may be configured to calculate deep tissue temperature using equations known to those skilled in the art. These equations may provide a mathematical framework that combines surface temperature measurements with radiometric temperature data to extrapolate deep tissue temperature conditions that may not be directly accessible through surface measurement techniques alone. The mathematical relationships may account for the thermal gradient that exists between surface tissue layers and deeper tissue regions, enabling calculation of temperature values at specific depths that correspond to target anatomical structures such as brain tissue or other internal organs where direct temperature measurement may not be practical or safe using conventional measurement approaches.

Surface temperature parameters may represent skin temperature measured by the thermistor that forms part of the sensor circuitry 300, providing direct measurement of surface temperature conditions at the sensor attachment location and establishing a baseline temperature reference corresponding to thermal conditions at the tissue-sensor interface. Radiometer temperature parameters may represent temperature determined according to a composite of temperature contributions from tissue layers within the field of view of the antenna 210, calculated as a weighted average temperature accounting for electromagnetic properties and attenuation characteristics of various tissue types encountered within the measurement volume. The skin temperature and radiometer temperature measurements serve as critical input parameters for the temperature calculation equations, with the skin temperature providing surface thermal conditions and the radiometer temperature providing deep tissue thermal information. The mathematical combination of these two temperature measurements enables accurate determination of deep tissue temperature using equations known to those skilled in the art. Various coefficients may be predetermined to account for the relationship between surface temperature gradients and deep tissue temperature characteristics for specific anatomical regions and patient populations, determined experimentally based on datasets that correlate surface temperature measurements, radiometric temperature data, and direct deep tissue temperature measurements obtained through invasive measurement techniques during controlled studies.

The temperature calculation methodology may incorporate calibration procedures that account for individual patient characteristics and measurement conditions that could influence the relationship between measured parameters and actual deep tissue temperatures. The calibration process may involve adjustment of calculation parameters based on patient-specific factors such as age, body composition, anatomical dimensions, and physiological conditions that affect heat transfer characteristics within the target tissue regions. The system may incorporate adaptive algorithms that modify the calculation parameters based on measurement history and comparison with reference temperature measurements obtained through alternative measurement techniques when available. The calibration approach may enable optimization of measurement accuracy for individual patients while maintaining the computational efficiency needed for real-time temperature monitoring applications where immediate feedback regarding deep tissue temperature conditions may be required for clinical assessment and treatment decisions. The skin temperature and radiometer temperature measurements provide the fundamental data inputs that enable the calculation equations to determine deep tissue temperature with enhanced accuracy compared to single-measurement approaches.

The integration of skin temperature measurements with radiometer temperature data may provide enhanced measurement accuracy compared to approaches that rely solely on surface temperature measurements or radiometric measurements alone. The combined measurement approach may compensate for limitations associated with individual measurement techniques while leveraging the advantages of both surface temperature sensing and microwave radiometry for deep tissue temperature determination. The mathematical combination of skin temperature and radiometer temperature measurements may enable extraction of deep tissue temperature information that accounts for thermal gradients and tissue property variations that could otherwise introduce errors in temperature calculations based on single measurement modalities. The integrated measurement approach may provide improved sensitivity to deep tissue temperature changes while maintaining stability against environmental variations and surface temperature fluctuations that could otherwise affect measurement accuracy during extended monitoring periods.

A number of implementations have been described. Nevertheless, it will be understood that various modifications may be made without departing from the spirit and scope of the disclosure. Accordingly, other implementations are within the scope of the following claims.

Embodiments of the present disclosure provide a sensor assembly for non-invasive temperature measurement using microwave radiometry. The assembly comprises a circuit board with a thermistor, capacitor, and inductor, where the inductor electrically isolates microwave signals from temperature sensing signals. A coaxial connector is positioned at one end of the circuit board with an antenna aperture at the opposite end. A shield layer extends over the circuit board with a sensor region, transition region, and extended region that are offset from the circuit board components. The shield layer is oriented at an angle relative to the circuit board. The inductor utilizes parasitic capacitance to improve antenna impedance matching, while the capacitor provides microwave signal isolation for the thermistor to prevent interference with signal detection.

## Claims

1. A sensor assembly for non-invasive temperature measurement, comprising:
a flexible circuit board having a first surface and a second surface opposite the first surface, the flexible circuit board including a dielectric layer, a first conductive layer disposed on the first surface, and a second conductive layer disposed on the second surface, wherein the first conductive layer includes an antenna aperture configured to receive microwave signals from tissue;
an inductor electrically connected to the antenna aperture;
a thermistor electrically connected to the inductor and configured to measure skin temperature;
a capacitor electrically connected between the thermistor and a ground connection;
a coaxial connector having a center conductor and an outer conductor, wherein the center conductor is electrically connected to the antenna aperture and the outer conductor is electrically connected to the ground connection;
a shield layer positioned above the first surface of the flexible circuit board and configured to reduce electromagnetic interference; and
an adhesive layer positioned on the second surface of the flexible circuit board and configured to adhere the sensor assembly to skin.

2. The sensor assembly of claim 1, wherein the flexible circuit board has a thickness of approximately 0.006 inches, and/or wherein the shield layer comprises aluminum foil having a thickness of approximately 0.002 inches.

3. The sensor assembly of claim 1 or 2, wherein the shield layer is oriented at an angle relative to the flexible circuit board such that vertices of the shield layer extend from sides of the antenna aperture to provide maximum shielding area in directions of highest antenna sensitivity to electromagnetic interference.

4. The sensor assembly of any of the preceding claims, further comprising a foam spacer positioned between the shield layer and the flexible circuit board, wherein the foam spacer comprises closed cell polyethylene foam.

5. The sensor assembly of any of the preceding claims, wherein the inductor has a self-resonant frequency, and wherein a parasitic capacitance of the inductor at the self-resonant frequency is configured to provide impedance matching for the antenna aperture.

6. The sensor assembly of any of the preceding claims, wherein the second conductive layer includes an aperture positioned beneath the center conductor of the coaxial connector, wherein the aperture is sized to optimize capacitance between the center conductor and the second conductive layer for impedance matching.

7. The sensor assembly of any of the preceding claims, wherein the coaxial connector comprises a push-on connector configured to rotate without disconnecting.

8. A radiometer system for measuring deep tissue temperature, comprising:
a sensor assembly including a flexible circuit board having an antenna aperture configured to receive microwave signals from tissue, an inductor electrically connected to the antenna aperture, a thermistor electrically connected to the inductor and configured to measure skin temperature, and a capacitor electrically connected between the thermistor and a ground connection;
a switch module electrically connected to the sensor assembly and configured to alternate between receiving signals from the sensor assembly and signals from a reference source;
a radiometer electrically connected to the switch module and configured to process the alternating signals to determine a temperature difference between tissue temperature and reference temperature; and
a temperature output calculator configured to calculate deep tissue temperature based on the temperature difference.

9. The radiometer system of claim 8, wherein the switch module comprises a reference sensor configured to measure ambient temperature and a reference heater configured to generate a reference signal at a controlled temperature, wherein the reference heater is preferably controllable to maintain a temperature approximately equal to an expected tissue temperature.

10. The radiometer system of claim 8 or 9, wherein the radiometer comprises a frequency mixer and an oscillator configured to convert received RF signals to an intermediate frequency for improved frequency selectivity and rejection of external RF interference, and/or wherein the radiometer further comprises a low noise amplifier, a band pass filter, an RF detector, a video amplifier, a synchronous detector, and a low pass filter.

11. The radiometer system of any of claims 8 to 10, wherein the temperature output calculator is configured to calculate deep tissue temperature based on at least a skin temperature and a radiometer temperature.

12. A method for non-invasively measuring deep tissue temperature, comprising:
positioning a sensor assembly on skin, the sensor assembly including an antenna aperture, a thermistor, an inductor electrically connecting the antenna aperture to the thermistor, and a capacitor electrically connected between the thermistor and ground;
receiving microwave signals from tissue through the antenna aperture;
measuring skin temperature using the thermistor while the inductor provides electrical isolation between microwave signals and temperature sensing signals;
alternating between processing signals from the sensor assembly and signals from a reference source using a switch module;
determining a temperature difference between tissue temperature and reference temperature using a radiometer; and
calculating deep tissue temperature based on the temperature difference.

13. The method of claim 12, wherein the step of alternating between processing signals from the sensor assembly and signals from a reference source comprises generating a reference signal using a reference heater maintained at a temperature approximately equal to an expected tissue temperature, wherein the reference heater is optionally controlled by a servo loop that automatically maintains the reference temperature within predetermined tolerance ranges.

14. The method of claim 12 or 13, wherein the step of determining a temperature difference comprises converting received RF signals to an intermediate frequency using a frequency mixer and an oscillator for improved frequency selectivity and rejection of external RF interference; and/or
wherein the step of calculating deep tissue temperature comprises calculating deep tissue temperature based on at least a skin temperature and a radiometer temperature.

15. The method of any of claims 12 to 14, wherein the sensor assembly includes a shield layer positioned above the antenna aperture and oriented at an angle relative to a flexible circuit board containing the antenna aperture such that vertices of the shield layer extend from sides of the antenna aperture to provide maximum shielding area in directions of highest antenna sensitivity to electromagnetic interference.
